# EUROPEAN PATENT APPLICATION

(11) **EP 1 048 299 A1**
(43) Date of publication of application: **02.11.2000**
(21) Application number: 99201350.8
(22) Date of filing: 28.04.1999
(51) Int. Cl.: A61K 39/395

(54) **Method for inhibiting cell functioning for use in anti-inflammatory and anti-tumour therapies**

(71) Applicant: Faculteit der Geneeskunde van de Vrije Universiteit, 1081 BT Amsterdam (NL)
(72) Inventor: Van den Berg, Timo Kars, 1081 BT Amsterdam (NL); Dijkstra, Christine Diederike, 1081 BT Amsterdam (NL)
(74) Representative: Hooiveld, Arjen Jan Winfried

(57) **Abstract**

The invention relates to a method for inhibiting cell functioning for use in anti-inflammatory and anti-tumour therapies in the body of a warm-blooded living being, which comprises administering to said being a drug comprising, in a quantity effective for said therapies, a substance that specifically recognizes the extracellular domain of SIRP (anti-SIRP substance) and that inhibits the functioning of pathologic myeloid cells.

The invention further relates to a drug to be used in the above method, and to the active substance of said drug.

## Description

The invention relates to a method for inhibiting cell functioning for use in anti-inflammatory and anti-tumour therapies in the body of a warm-blooded living being. The invention further relates to a drug to be used in the above method, and to the active substance of said drug.

Inflammations in the body of a warm-blooded living being, in particular a human being, cause many diseases and disorders, and may even turn out to be life-threatening. Therefore, for many decades already it is a major challenge to the clinician to find an effective therapy in treating inflammatory diseases. Various inflammatory diseases, such as rheumatoid arthritis, multiple sclerosis, glomerulonephritis, diabetes and asthma, are the result of unwanted immune responses. As described, for instance, in a recent survey entitled "Manipulation of the Immune Response" ("Immunobiology", 3rd Edition; C.A. Janeway, P. Travers; publ. Current Biology/ Garland/ Churchill Livingstone 1997; Chapter 13), current treatments for immunological disorders are nearly all empirical in origin using immunosuppressive drugs identified by screening large numbers of natural and synthetic compounds.
According to this survey, these drugs may be divided into three categories, viz. (i) drugs of the corticosteroid family, (ii) cytostatic drugs, and (iii) fungal and bacterial derivatives. In this survey it is noted, that these drugs are all very broad in their actions and inhibit protective functions of the immune system as well as harmful ones. In fact, the ideal immunosuppressive agent would be a drug that targets the specific part of the immune response responsible for causing the relevant tissue injury. Consequently, according to this survey, antibodies themselves, by virtue of their exquisite specificity, may offer the best possibility for the therapeutic inhibition of specific immune responses. Such immunosuppressive monoclonal antibodies can act by inhibiting target cell functioning. Their promising potential in immunosuppression has already been established. However, as yet these antibodies are not widely and generally used as anti-inflammatory drugs, largely due to the fact that the appropriate targets have not been identified.

It is the objective of the present invention to provide a method for inhibiting or controlling target cell functioning, for use in anti-inflammatory and anti-tumour therapies in the body of a warm-blooded living being by administration of a drug, having superior therapeutic properties compared to existing anti-inflammatory and anti-tumour drugs. Various requirements should be imposed on a drug to be used in such therapies, for example, non-toxic, no adverse influence on the host resistance, and highly selective to avoid burdening of non-target tissues and organs with drug material.

According to the present invention the above-defined objective can be achieved by a method which comprises administering to said being a drug comprising, in a quantity effective for said therapies, a substance that specifically recognizes the extracellular domain of SIRP (= signal regulatory protein) (anti-SIRP substance) and that inhibits the functioning of pathologic myeloid cells. By using a drug according to the method of the present invention, both a highly selective and an effective therapy in treating inflammatory diseases, in particular autoimmune diseases and allergies, and tumours can be achieved.

Well-known examples of myeloid cells are macrophages, which are continuously replinished from a population of dividing and maturing myeloid precursor cells in the bone marrow. This ensures the continuous availability of macrophages in all tissues of the body and allows a fast and efficient response in case of infections. In a number of circumstances, however, macrophages do not play a benificial role. In a variety of autoimmune diseases, like rheumatoid arthritis, multiple sclerosis, glomerulonephritis etc., and allergies, like asthma, activated macrophages play an important role in the induction and/or maintenance of inflammations that, as a consequence, forms the basis for the (general chronical) clinical symptoms.
In addition, under certain circumstances the myeloid precursor cells may cause pathologies; the unlimited growth of these myeloid precursor cells is the cause of certain malignant tumours, in particular myeloid leukemia.

More in particular, said anti-SIRP substance to be used in the method of the present invention is characterized in that it inhibits the functioning of macrophages by suppressing their activation by a factor of at least 10 as measured by each of the following so-called macrophage activity tests: (i) the production of nitric oxide (NO), (ii) the production of reactive oxygen species, in particular superoxides (e.g. H₂O₂), and (iii) the production of tumour necrosis factor - alpha (TNF).
The above tests for measuring the activity of macrophages are described in detail in Example II hereinafter. It has been found, that the substances to be used according to the method of the invention show the above striking effect in all three above tests. Therefore these tests are a convenient tool of distinguishing substances within the scope of the invention from other compounds.

Signal-regulatory proteins (SIRP), as recently disclosed by S. Adams et al. in J. Immunol. 161: 1853-1859 (1998), are transmembrane glycoproteins, inhibiting signaling through receptor tyrosine kinases but having a physiological function which is unknown up to the present. SIRP is selectively expressed on the surface of myeloid cells, such as macrophages, monocytes, granulocytes and dendritic cells, and neurons. The active substances to be used in the method of the present invention can closer be defined as proteinaceous substances, i.e. substances having a polypeptide main-chain or backbone. According to the present invention these closer-defined substances should meet the following requirements:
(1) specific recognition of (the extracellular domain of) SIRP, according to a test method as disclosed in the above publication by Adams et al.; and
(2) suppression of the activation of macrophages according to the three tests as described above.

As mentioned above, the functioning of myeloid precursor cells may cause pathologies; an unlimited growth or division of myeloid precursor cells is the cause of myeloid leukemia, a malignant tumour. It has been found that the above anti-SIRP substances to be used according to the method of the invention can also inhibit the functioning of pathologic myeloid cells by strongly suppressing the division of macrophage tumour cell lines. More specifically, the suppression of this cell division is also found to amount to a factor of at least 10, as demonstrated in the so-called macrophage division test (Examples II).
Therefore, a drug comprising said anti-SIRP substance in an effective quantity can successfully be used in anti-tumour therapy, in particular for treating myeloid leukemia, because the selective binding of these substances to the extracellular domain of SIRP can effectively and selectively control the division of myeloid cells.

The above-mentioned functioning of myeloid cells, in particular macrophages, encompasses not only their activation and division, but also the phenomenon of phagocytosis, that is the uptake of other organisms or other particles.
In case of gene-targeted therapies, e.g. gene-targeted anti-tumour therapy, where genes packed in vector particles (vehicles) are targeted to different cells or tissues, macrophages with their potent phagocytic capacity are a major barrier in the efficient delivery of the genes of interest. The method of the present invention is to be considered to also encompass a method for use in such gene-targeted therapies. If these therapies are attended by a drug comprising the above anti-SIRP substance, a common pathway of macrophage phagocytosis can be inhibited, resulting in a temporal suppression of said phagocytosis and consequently in a considerable improvement of the efficacy of these therapies. This unique property in gene-targeted therapies makes the active substances according to the present invention extremely useful therapeutically: see the results of the so-called macrophage phagocytosis test in Example II.

More specifically, the anti-SIRP substances to be used according to the method of the present invention, can be characterized in a preferred embodiment as being selected from the group consisting of Fab-fragments of monoclonal antibodies and (bio)chemically modified products of such fragments wherein the intended anti-SIRP activity has been maintained. Suitable examples of such modified products of said Fab-fragments are NH-acylated products, S-S - reduced products comprising free mercapto groups, etc., provided that the intended activity has been maintained.

It has been found, that Fab-fragments of the monoclonal antibodies ED9 and ED17, as well as the above-mentioned modified products thereof, are extremely promising for the therapeutic method of the invention, as can be concluded from a number of suitable cell culture experiments that are predictive for human application. These experiments are described in the accompanying Examples. From the results of these experiments it will be evident, that the tested anti-SIRP substances have properties, which make them particularly suitable for use in the method of the invention.

The above monoclonal antibodies ED9 and ED17 are described in the above-mentione publication by Adams et al., as well as their selective recognition of rat SIRP (anti-SIRP activity) that is selectively expressed by myeloid cells, e.g. by macrophages. These authors have found, that the binding of these monoclonal antibodies to macrophages induces the production of nitric oxide (NO). It is indeed quite a surprise, that the inventors of the present invention have found, that the Fab-fragments of the same monoclonal antibodies ED9 and ED17 show an opposite effect after binding to macrophages, viz. a suppression of the production of nitric oxide. It is precisely this effect that makes the anti-SIRP substances of the present invention so suitable for the intended use.

The present invention also relates to a drug for inhibiting cell functioning for use in anti-inflammatory and anti-tumour therapies, as indicated above. Such a drug according to the present invention comprises, in addition to a pharmaceutically acceptable carrier and, if desired, one or more pharmaceutically acceptabe adjuvants, as the active ingredient an anti-SIRP substance that inhibits the functioning of pathologic myeloid cells. The above-mentioned solid or liquid carriers, as well as the suitable adjuvants are well-known in pharmacy.
In a preferred embodiment said drug according to the present invention comprises an anti-SIRP substance selected from the group consisting of Fab-fragments of monoclonal antibodies, preferably of ED9 or ED17, and (bio)chemically modified products of such fragments wherein the intended anti-SIRP activity has been maintained.

Finally the present invention relates to an anti-SIRP substance that inhibits the functioning of pathologic myeloid cells, said anti-SIRP substance being selected from the group consisting of Fab-fragments of monoclonal antibodies, preferably of ED9 or ED17, and (bio)chemically modified products of such fragments wherein the intended anti-SIRP activity has been maintained.

The invention will now be described in greater detail with reference to the following specific Examples.

### Example I

### Preparation of Fab-fragments of ED9 and ED17.

The starting monoclonal antibodies ED9 and ED17 are disclosed by Damoiseaux et al. in J. Leukocyte Biol. 46:556-564 (1989) and 49: 434-441 (1991). The Fab-fragments of these antibodies are obtained by papain-protolytic digestion. For this purpose a papain-solution, containing 0.1 mg of papain per ml PBS buffer solution (0.02M EDTA and 0.02M cystein in PBS), is added to the same volume of a solution of the antibody (1 mg/ml) in PBS. The mixture is incubated at 37°C, and after a certain time, determined by making a time-series, the reaction is stopped by adding a 0.03M iodoacetamide solution (addition of 20 µl 0.3M iodoacetamide to 110 µl incubated mixture) . The mixture is now dialysed against 2 l PBS at pH 8.0, 0/N at 4°C. The solution is chromatographed over a protein A sepharose column, concentrated to 5 ml at reduced pressure, and chromatographed over a superose 12 column. The fractions of 50 kD are received and purity-controlled on non-reduced SDS-PAGE^{R}. The solution of the Fab-fragments ED9 and ED17, so obtained, are used as such in the cell culture experiments described in Example II.

### Example II

### Cell culture experiments

### The macrophage activity test

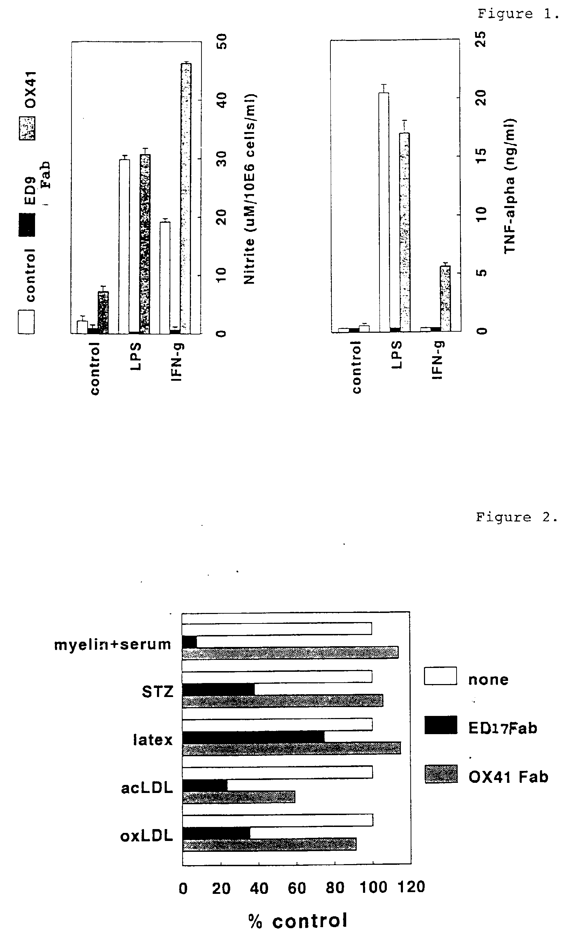

Rat peritoneal macrophages, obtained by peritoneal lavage, of the rat macrophage cell line NR8383 (Adams et al. 1998) are cultured at a density of 0.25 x 10⁶ cells/ml in RPMI-1640 medium containing 2% fetal calf serum and 2 mM glutamine, 100 U/ml penicillin and 100 µg/ml streptomycin. Macrophage activating stimuli (100 ng/ml lipopolysaccaride (LPS), or 20 U/ml gamma-interferon (IFN)-g) are added in the presence (or absence) of anti-SIRP Fab-fragments (ED9 or ED17; 40 µg/ml) or control Fab-fragments (OX41, Adams et al. 1998; 40 µg/ml). After 18-20 hours the cell culture supernatants (separated from the cells by centrifugation for 7 min. at 500g) are harvested. NO production in supernatants is measured using Griess reagent (Ding et al. (1988), J. Immunol. 141:2407) using NaNO2 to produce a calibration curve. TNFα, IL1β and IL6 are measured by enzyme-linked immunosorbent assay as described (Vincent et al. (1996), Glia 17:94; Lenczowski et al. (1997), Am.J.Physiol. 273:R1870). The results are presented in the diagram of Figure 1.

### The macrophage phagocytosis test

0.5 x 10⁶ rat peritoneal macrophages are plated in each well of a 24-well cell culture plate in RPMI-1640 medium containing 10% fetal calf serum and 2 mM glutamine, 100 U/ml penicillin and 100 µg/ml streptomycin, and are then allowed to adhere for 1-1.5 hours at 37°C in a 5% CO₂ atmosphere. After this the cells are washed 2x and incubated with 0.5 ml HEPES (25 mM) -buffered RPMI containing 2 µg oxidated LDL (low-density lypoproteins), 2 µg acetylated LDL (both: FITC-labelled; Molecular Probes), 1 µl latex beads (FITC-labelled; Molecular Probes), 2 µg serum treated zymosan (FITC-labelled), or rat myelin (DiI-labelled) plus 5% fresh rat serum. These incubations are performed in the presence (or absence) of anti-SIRP Fab-fragments (ED9 or ED17; 40 µg/ml) or control Fab-fragments (OX41; 40 µg/ml). After 1.5 hours the cells are washed to remove non-bound particles, cells are detached by incubation in 5 mM EDTA in PBS and mean fluorescence intensity for each cell is measured on a FACScan^{R}. Values are plotted as the percentage of control phagocytosis: Figure 2.

FITC and DiI are fluorescent dyes, well-known in the art.

### The macrophage division test

The rat macrophage cell line NR8383 (Adams et al. 1998) are cultured at a density of 0.25 x 10⁶ cells/ml in a 96-well cell culture plate in RPMI-1640 medium containing 2% fetal calf serum and 2 mM glutamine, 100 U/ml penicillin and 100 µg/ml streptomycin. This is performed in the presence (or absence) of anti-SIRP Fab-fragments (ED9 or ED17; 40 µg/ml) or control Fab-fragments (OX41; 40 µg/ml). After 24 h ³H-thymidine (1 µCi/well) is added and the cells are incubated for another 6 hours. The cells are harvested using a cell harvester and cell incorporated radioactivity is determined in a Micro-β-plate reader. The mean results are shown in Table 1 below:

| Treatment | Mean (in c.p.m.) | SD (standard dev.) |
|---|---|---|
| control | 132783 | 2730 |
| ED17 Fab | 6845 | 197 |
| OX41 Fab | 154889 | 8528 |

### Results

In all above experiments the results of ED9 Fab and of ED17 Fab are comparable with each other; therefore the results presented are confined to one active substance.
To evaluate the effects of ED9 or ED17 Fab-fragments, cell culture experiments using animal cells, predictive for human myeloid and/or inflammatory cells, are performed. In the macrophage activity test (Figure 1) the effect on the production of the inflammatory mediators reactive oxygen species (H₂O₂ as ROS), nitric oxide (NO) and the proinflammatory cytokine TNFa is measured. As can be seen, ED9 Fab strongly suppresses the production of ROS(not shown), NO and TNFα, whereas irrelevant OX41 Fab-fragments do not have this effect.
To evaluate the effect of ED9 or ED17 Fab on phagocytosis peritoneal macrophages are assayed as described in the macrophage phagocytosis test. As can be seen (Figure 2), ED17 Fab strongly suppresses the phagocytosis of various particles, including myelin+serum, serum-treated zymosan, latex beads, and oxydated- or acetylated-low density lipoproteins. Again control OX41 Fab fragments had no such effects.
To examine the effects of ED9 or ED17 Fab on myeloid cell division, NR8383 cells are assayed as described in the macrophage division test. As illustrated in table 1, ED17 strongly inhibits division (analyzed by thymidine incorporation), whereas OX41 Fab has little effect.

### Discussion and Conclusion

Taken together, these results show that Fab fragments of antibodies ED9 or ED17, directed against an overlapping epitope of SIRPα (Adams et al. 1998), can potently suppress the activation and phagocytosis of macrophages and the cell division of myeloid cells. No such effects are seen with Fab fragments of other antibodies, directed against a different SIRPα epitope (OX41). These properties make the extracellular domain of SIRPα a target for: (1) anti-inflammatory therapy and (2) anti-tumour therapy of myeloid leukemia. In addition, (3) temporal suppression of phagocytosis via SIRPα ligation can help to increase the efficiency of gene therapy.

Figure 1. Anti-SIRPα Fab fragments (ED9) inhibit the production of (a) NO and (b) TNFα induced by LPS (100 ng/ml) or IFN-γ (20 U/ml) in NR8383 macrophages.

Figure 2. Anti-SIRPα Fab fragments (ED17) inhibit the phagocytosis by peritoneal macrophages of various particles, including myelin+serum, serum-treated zymosan, latex beads, and oxydated- or acetylated-low density lipoproteins.

Table 1. Anti-SIRPα Fab fragments (ED17) inhibit the division of myeloid NR8383 cells.

## Claims

1. A method for inhibiting cell functioning for use in anti-inflammatory and anti-tumour therapies in the body of a warm-blooded living being, which comprises administering to said being a drug comprising, in a quantity effective for said therapies, a substance that specifically recognizes the extracellular domain of SIRP (anti-SIRP substance) and that inhibits the functioning of pathologic myeloid cells.

2. The method as claimed in claim 1, wherein said substance inhibits the functioning of macrophages by suppressing their activation by a factor of at least 10 as measured by each of the following macrophage activity tests: (i) the production of nitric oxide (NO), (ii) the production of reactive oxygen species, and (iii) the production of tumour necrosis factor - alpha (TNF-α).

3. The method as claimed in claim 1, wherein said substance inhibits the functioning of pathologic myeloid cells by suppressing the division of macrophage tumour cell lines by a factor of at least 10 as measured by the macrophage division test.

4. The method as claimed in any of claims 1-3 for treating pathologies selected from inflammations, caused by autoimmune diseases or by allergies, and myeloid leukemia.

5. The method as claimed in claim 1, wherein said substance inhibits the functioning of macrophages by temporally suppressing their phagocytosis as measured by the macrophage phagocytosis test.

6. The method as claimed in claim 5 for improving the efficacy of gene-targeted therapies.

7. The method as claimed in any of the preceding claims, characterized in that said anti-SIRP substance is selected from the group consisting of Fab-fragments of monoclonal antibodies and (bio)chemically modified products of such fragments wherein the intended anti-SIRP activity has been maintained.

8. The method as claimed in claim 7, wherein said anti-SIRP substance is a Fab-fragment of monoclonal antibody ED9 or ED17, or said modified product thereof.

9. Use of a substance, that specifically recognizes the extracellular domain of SIRP (anti-SIRP substance) and that inhibits the functioning of pathologic myeloid cells, for the manufacture of a drug for inhibiting cell functioning for use in anti-inflammatory and anti-tumour therapies.

10. The use as claimed in claim 9, wherein the anti-SIRP substance is selected from the group consisting of Fab-fragments of monoclonal antibodies, preferably of ED9 or ED17, and (bio)chemically modified products of such fragments wherein the intended anti-SIRP activity has been maintained.

11. A drug to be used according to any of the preceding claims, comprising, in addition to a pharmaceutically acceptable carrier and, if desired, one or more pharmaceutically acceptable adjuvants, as the active substance an anti-SIRP substance that inhibits the functioning of pathologic myeloid cells.

12. A drug as claimed in claim 11, wherein the anti-SIRP substance is selected from the group consisting of Fab-fragments of monoclonal antibodies, preferably of ED9 or ED17, and (bio)chemically modified products of such fragments wherein the intended anti-SIRP activity has been maintained.

13. An anti-SIRP substance that inhibits the functioning of pathologic myeloid cells, selected from the group consisting of Fab-fragments of monoclonal antibodies, preferably of ED9 or ED17, and (bio)chemically modified products of such fragments wherein the intended anti-SIRP activity has been maintained.
